# EUROPEAN PATENT APPLICATION

(11) **EP 1 897 534 A2**
(43) Date of publication of application: **12.03.2008**
(21) Application number: 07005891.2
(22) Date of filing: 22.03.2007
(51) Int. Cl.: A61K 8/81, A61Q 1/10

(54) **Cosmetic or dermatologic compositions containing microspheres**

(30) Priority: 08.08.2006 US 500344
(71) Applicant: L'Oréal, 75008 Paris (FR)
(72) Inventor: Quadir, Murat, Scotch Plains, New Jersey 07076 (US); Burakov, Dina, Edison, New Jersey 08837 (US); Rollat-Corvol, Isabelle, 75017 Paris (FR)
(74) Representative: Le Coupanec, Pascale A.M.P.

(57) **Abstract**

The invention relates to cosmetic or dermatologic compositions comprising a microsphere and a film forming component.

## Description

The present invention generally relates to cosmetic or dermatologic compositions comprising a microsphere and a film forming component. Such compositions possess improved cosmetic or dermatologic properties and characteristics such as, for example, increasing eyelash length and/or volume, improving the hold of the shape or curl of hair/eyelashes, permitting hair or eyelashes to be reshaped into the original style without reapplying a hair or eyelash styling composition and/or heat, or increasing transfer-resistance, waterproofing and/or long-wear properties.

Fixing keratin materials such as hair and eyelashes in place is an important element in styling, and this generally involves either fixing a shape that has already been styled, or simultaneously shaping and fixing of the keratin material. While styling compositions currently on the market are generally effective in holding the keratin material in a desired shape or configuration, many are not designed to allow the style to be later modified to a desired shape or reshaped into the original style without reapplying a styling composition and/or heat. Moreover, under various kinds of stress, styles resulting from the use of conventional products have a tendency to take on an undesirable permanent set, which cannot easily be modified.

U.S. Patent No. 6,548,051 and U.S. patent application publication nos. 2002/0059941 and 2006/0024255 address such shortcomings of traditional styling composition by providing reshapable hair styling compositions.

While there have been pioneering advances in hair styling products by the introduction of such reshapable hair care systems, further improvement remains desirable. Further advances in adjusting the degree of adhesion and improving the overall look, feel and manageability of hair are still worthy, if not lofty goals.

Accordingly, one aspect of the present invention is a care and/or makeup and/or treatment composition for keratin materials such as hair, eyelashes, skin, lips and nails which is able to address or overcome at least some of the problems generally associated with cosmetic or dermatologic compositions such as, for example, not allowing for reshapability, poor transfer-resistance or adhesion, poor hold characteristics, etc.

The present invention relates to composition, in particular cosmetic or dermatologic compositions comprising a microsphere and a film forming component.

In particular, the present invention relates to compositions cosmetic or dermatologic compositions comprising in a cosmetically acceptable medium, an adhesive microsphere, and a film forming component.

The present invention relates to cosmetic or dermatologic compositions which are free of, essentially free of or substantially free of non-adhesive particles comprising a microsphere and a film forming component.

The present invention also relates to compositions for eyelashes such as mascaras, topcoats and basecoats comprising a microsphere and a film forming component.

The present invention also relates to compositions for hair such as sprays, mousses, styling gels, leave-on conditioners, shampoos, conditioners, permanent waving compositions, hair care products, hair treatment products, and hair styling products comprising a microsphere and a film forming component.

The present invention also relates to compositions for skin or lips such as a foundation, lipstick, liquid lip color, topcoat or basecoat comprising a microsphere and a film forming component.

The present invention also relates to methods of improving performance properties of compositions for keratin materials such as, for example, transfer-resistance, long-wear and/or waterproofing, comprising combining in a cosmetic or dermatologic composition a microsphere and a film forming component.

The present invention also relates to methods of increasing eyelash or hair volume, strength and/or length comprising applying to eyelashes or hair an eyelash or hair volume-, strength and/or length-increasing effective amount of a composition comprising a microsphere and a film forming component.

The present invention also relates to methods of styling a keratin material such as hair or eyelashes comprising applying to the keratin material a keratin material styling effective amount of a composition comprising a microsphere, in particular an adhesive microsphere, and a film forming component.

The present invention also relates to methods of restyling, reshaping or repeatedly fixing a keratin material such as hair or eyelashes comprising initially applying to the keratin material a keratin material styling effective amount of a composition comprising a microsphere and a film forming component, and subsequently restyling, reshaping or repeatedly fixing the keratin material without reapplying the composition to the keratin material.

The present invention also relates to methods of holding the shape or curl of a keratin material such as hair or eyelashes comprising applying to the keratin material a shape or curl holding effective amount of a composition comprising a microsphere, in particular an adhesive microsphere, and a film forming component.

The present invention further relates to methods of making-up keratin materials such as eyelashes, nails, skin or lips comprising applying an eyelash, nail, skin or lip making-up effective amount of a composition comprising a microsphere and a film forming component, to eyelashes, nails, skin or lips in need of such making-up.

The present invention also relates to methods, in particular non therapeutic methods, of treating or caring for keratin materials such as eyelashes, nails, hair, skin or lips by applying compositions of the present invention to the eyelashes, nails, hair, skin or lips in an amount sufficient to treat and/or care for the eyelashes, nails, hair, skin or lips.

The present invention further relates to methods of enhancing the appearance of keratin materials such as eyelashes, nails, hair, skin or lips by applying compositions of the present invention to the eyelashes, nails, hair, skin or lips in an amount sufficient to enhance the appearance of the eyelashes, nails, hair, skin or lips.

The present invention relates to the use of an adhesive microsphere and a film forming component to prepare a cosmetic and/or dermatologic composition for styling a keratin material and/or holding the shape or curl of a keratin material.

In certain embodiments, the present invention facilitates imparting at least one desirable property such as, without limitation, reduced tactile stickiness, luster, shine, silky feel or appearance to keratin materials. ln a particularly preferred embodiment, the present invention provides formulations that facilitate imparting at least one desirable property to keratin materials like hair and eyelashes such as, without limitation, reduced tactile stickiness, luster, shine, silky feel and/or appearance, while substantially maintaining the keratin material's fixability and reshapability.

lt is to be understood that both the foregoing general description and the following detailed description are exemplary and explanatory only, and are not restrictive of the invention.

As used herein, the expression "at least one" means one or more and thus includes individual components as well as mixtures/combinations.

"Cosmetically acceptable medium" means a medium that is compatible with any keratin material, such as the skin, the hair, the nails, the eyelashes, the eyebrows, the lips and any other area of body or facial skin.

"Transfer resistance" as used herein refers to the quality exhibited by compositions that are not readily removed by contact with another material, such as, for example, a glass, an item of clothing or the skin, for example, when eating or drinking. Transfer resistance may be evaluated by any method known in the art for evaluating such. For example, transfer resistance of a composition may be evaluated by a "kiss" test. The "kiss" test may involve application of the composition to human lips followed by "kissing" a material, for example, a sheet of paper, after expiration of a certain amount of time following application, such as 2 minutes after application. Similarly, transfer resistance of a composition may be evaluated by the amount of product transferred from a wearer to any other substrate, such as transfer from the neck of an individual to a collar after the expiration of a certain amount of time following application. The amount of composition transferred to the substrate (e.g., collar, or paper) may then be evaluated and compared. For example, a composition may be transfer resistant if a majority of the product is left on the wearer, e.g., lips, neck, etc. Further, the amount transferred may be compared with that transferred by other compositions, such as commercially available compositions.

"Long wear" compositions as used herein, refers to compositions where at least one property chosen from consistency, texture, and color remains the same as at the time of application, as viewed by the naked eye, after an extended period of time, such as, for example, 1 hour, 2 hours, and further such as 8 hours. Long wear properties may be evaluated by any method known in the art for evaluating such properties. For example, long wear may be evaluated by a test involving the application of a composition to human skin (including lips) and evaluating the consistency, texture and color of the composition after an extended period of time. For example, the consistency, texture and color of a lip composition may be evaluated immediately following application and these characteristics may then be re-evaluated and compared after an individual has worn the lip composition for a certain amount of time. Further, these characteristics may be evaluated with respect to other compositions, such as commercially available compositions.

"Waterproof" as used herein refers to the ability to repel water and permanence with respect to water. Waterproof properties may be evaluated by any method known in the art for evaluating such properties. For example, a mascara composition may be applied to false eyelashes, which may then be placed in water for a certain amount of time, such as, for example, 20 minutes. Upon expiration of the pre-ascertained amount of time, the false eyelashes may be removed from the water and passed over a material, such as, for example, a sheet of paper. The extent of residue left on the material may then be evaluated and compared with other compositions, such as, for example, commercially available compositions. Similarly, for example, a composition may be applied to skin, and the skin may be submerged in water for a certain amount of time. The amount of composition remaining on the skin after the pre-ascertained amount of time may then be evaluated and compared. For example, a composition may be waterproof if a majority of the product is left on the wearer, e.g., eyelashes, skin, etc.

"Tackiness" as used herein refers to measuring the maximum tensile force, Fₘₐₓ, required while separating two surfaces. Depending on the application envisaged and the formulation being designed, the desirable value for Fₘₐₓ may vary. In some embodiments, the substantially non-tacky compositions have a Fₘₐₓ of less than about 4 Newton (N), less than about 1 N, less than about 0.5 N, less than about 0.3 N, less than about 0.2 N or less than 0.1 N. One of ordinary skill in the art can determine the Fₘₐₓ of the composition by, for example, determining the maximum force of traction, measured with an extensiometer of the LLOYD model LR5K type, needed to detach two surfaces.

For example, two 38 mm² surfaces, A and B, which are solid, rigid, inert, and non-absorbing, are mounted on movable mounts, facing each other. The surfaces may be movable either toward or away from each other, or one may move surface A independently from surface B or vice versa. Prior to insertion into the extensiometer, surface A is coated with the composition to be measured, which may be dissolved in a solvent such as aqueous, hydroalcoholic, hydrocarbon, silicone, and alcoholic solvents in a concentration of from about 10 to about 30%, preferably 20%, the surface A is coated in a thickness of from 1 to 10 mil, preferably 1 mil, and the surface is dried for 24 hours at room temperature, e.g., 22 to 25°C, at a relative humidity of about 50%. Once inserted in the extensiometer, surface A is subjected for 20 seconds to a compression force of 3 N against surface B and then subjected for 30 seconds to tensile force at a rate of 20 mm/minute. The amount of force, Fₘₐₓ, needed to obtain initial separation is then noted. A mean Fₘₐₓ is determined by carrying out the procedure with multiple pairs, preferably at least six pairs, of surface A and surface B.

The term "reshapable" means to provide a hairstyle (fixability) and/or hold that can be restored or modified without additional material or heat being applied. This does not mean that additional product and/or heat may not be applied. It may be desirable to add heat and/or additional styling compositions in terms of speed, ease of use, if hair becomes unduly wet or dirty, is excessively combed, brushed or manipulated, washed, or when hair is to be dramatically restyled. At least some of the compositions in accordance with the invention should be reshapable, as judged by a professional hair stylist of ordinary skill, for at least 4 hours and up to 24 hours or more after initial application. Preferably and merely for example, in order to restore or modify the hairstyle in case of "drooping" or loss of setting (dishevelment), no new materials, such as water or any form of fixing agent, or heat are required. Other terms, which may be synonymous with reshapable, include repositionable, remoldable, restyleable, rearrangeable, and remodellable. The term "reshapable" also means to provide a hairstyle that can retain or hold a desired shape or configuration until water, heat, time and/or physical contact destroys the desired shape or configuration.

The cosmetic compositions and methods of the present invention can comprise, consist of, or consist essentially of the essential elements and limitations of the invention described herein, as well as any additional or optional ingredients, components, or limitations described herein or any otherwise useful ingredient found in personal care compositions intended for application to keratin materials.

The composition of the present invention may be in any form. For example, it may be a paste, a solid or a cream. The composition of the invention may be transparent or clear, including for example, a composition without pigments. The composition can also be a molded composition or cast as a stick or a dish. The composition in one embodiment is a solid such as a molded stick or a poured stick. The compositions of the present invention may also be in the form of a lip composition such as a lipstick or a liquid lip color, a foundation, a mascara product, a hair gel, a hair mousse, or a hair spray (aerosol or non-aerosol).

As defined herein, stability is tested by placing the composition in a controlled environment chamber for 8 weeks at 25°C. In this test, the physical condition of the sample is inspected as it is placed in the chamber. The sample is then inspected again at 24 hours, 3 days, 1 week, 2 weeks, 4 weeks and 8 weeks. At each inspection, the sample is examined for abnormalities in the composition such as phase separation if the composition is in the form of an emulsion. The stability is further tested by repeating the 8-week test at 40°C, 37°C, 45°C, 50°C and/or under freeze-thaw conditions. A composition is considered to lack stability if in any of these tests an abnormality that impedes functioning of the composition is observed. The skilled artisan will readily recognize an abnormality that impedes functioning of a composition based on the intended application.

Microsphere

According to the present invention, compositions comprising at least one microsphere are provided. Preferably, the microspheres have adhesive properties and/or are not film forming agents. Most preferably, the microspheres have adhesive properties and are not film forming agents. Suitable microspheres include but are not limited to Gel Tac microspheres (available from API) such as, for example, those found in Gel Tac commercial products Gel Tac 100 A, Gel Tac 100 B, Gel Tac 100 C, Gel Tac 100 K and Gel Tac 205D, as well as those microspheres disclosed in U.S. patent application publication nos. 2006/0024255 and 2002/0041858.

The microspheres of the present invention are preferably prepared via suspension polymerization of one or more free radical polymerizable monomers in the presence of surfactants, polymerization initiators and/or suspension stabilizers. Most preferably, the microspheres are prepared by suspension polymerization of acrylic monomers in the presence of a polymerization initiator. Suitable methods of preparing the microspheres are disclosed in U.S. Pat. No. 3,691,140, U.S. Pat. No. 4,166,152, U.S. Pat. No. 4,495,318, U.S. Pat. 4,598,112, U.S. Pat. No. 4,786,696, U.S. Pat. No. 4,839,416, U.S. Pat. No. 4,968,562, U.S. Pat. No. 5,045,569, U.S. Pat. No. 5,571,617, U.S. Pat. No. 5,409,977, U.S. Pat. No. 5,656,705, U.S. Pat. No. 5,719,247 and/or U.S. patent application publication no. 2003/0109630.

Preferably, monomers should be chosen to yield microspheres having a glass transition temperature (Tg) below -20°C. Such monomer choice provides the microspheres with improved adhesive properties.

According to preferred embodiments, the microspheres are prepared by homo- or co-polymerization of monomer(s) selected from the group consisting of acrylates, methacrylates, acrylic acid, methacrylic acid, acrylamides, acrylonitriles, pyrrolidones, vinyl esters, and mixtures thereof, and in particular selected from the group consisting of acrylates, methacrylates, acrylic acid, methacrylic acid, and mixtures thereof. For example, monomers which may be used to prepare the microspheres include, but are not limited to, alkyl acrylates such as butyl acrylate, ethyl acrylate, methyl acrylate, and 2-ethylhexylacrylate, alkyl methacrylates such as methyl methacrylate and butyl methacrylate, polar comonomers such as acrylic acid, methacrylic acid, hydroxyethyl acrylate, hydroxypropyl acrylate, N-tertiary octylacrylamide, acrylonitrile, acrylamide, 1-vinyl-2-pyrrolidone, sodium vinyl sulfonate, vinyl esters such as vinyl acetate, vinyl propionate, vinyl neodecanoate, vinyl stearate and vinyl pivalate. For example, the monomer is ethylhexylacrylate. Cross-linking monomers such as, for example, butanediol diacrylate and hexanediol diacrylate can also be used. Other useful monomers and combinations thereof which can be used in the practice of the invention would be apparent to one skilled in the art.

Polymerization initiators useful in microsphere polymerization include, but are not limited to, dialkyl peroxides such as lauroyl peroxide (Laurox from Akzo Nobel), diacyl peroxides such as dibenzoyl peroxide (Lucidol A75 from Elf Atochem), peroxyesters such as t-amyl peroxypivalate (Lupersol 554-M50 from Elf Atochem), t-butyl peroctoate (Luperox 26 from Elf Atochem and Trigonox 21 from Akzo Chemicals), azo compounds such as 2,2'-azobis(isobutyronitrile) (VAZO 64) and 2,2'-azobis(2-methylbutanenitrile) (VAZO 67), and 2,2'-azobis(2,4-dimethylpentanenitrile) (VAZO 52), all from DuPont. Other useful initiators and combinations thereof which can be used in the practice of the invention would be apparent to one skilled in the art.

According to particularly preferred embodiments of the present invention, 2-ethyl hexyl acrylate (monomer) and t-butyl peroctoate (as initiator) are used to prepare the micropsheres as described in U.S. patent application publication no. 2003/0109630.

Microspheres of the present invention may be of widely varied shapes such as spheres, spheroids, platelets, and irregularly shaped particles. The size of the micropshere is usually described in terms of its length and/or width. ln the context of the present invention, "length" of the microsphere is intended to mean the maximum distance possible to be measured by appropriate microscopy techniques, between two opposing points of the microsphere. The "width" of the microsphere is intended to mean the minimum distance possible to be measured by appropriate microscopy techniques, between two opposing points of the microsphere.

In one embodiment, the microspheres have a ratio of length to width of greater than or equal to about 1:1.

In another embodiment, the microspheres have a mean length of about 1 to about 1000 microns, preferably from 1 to less than 30 microns, preferably from 30 to 1000 microns, preferably from 30 to 100 microns, and more preferably from about 30 to about 50 microns. The mean length of the microspheres may be measured by the following methods: optical microscopy, sieving, sedimentation, radiant diffusion, absorption, Coulter's principle, laser light diffraction, X-ray, and Sensing-zone method, which are described by Clyde Orr, "Size Measurement of Particles," in Kirk-Othmer Encyclopedia of Chemical Technology, vol. 21, pp. 106-131 (3rd ed. 1983) and by Terry Allen, "Analyse et Caracterisation, Technique de l'ingenieur" in Etudes de Structure-Granulometrie, vol.12, pp.1040-9 to 1040-26 (1996). In one embodiment, optical microscopy is used to determine the mean length of the microsphere.

Preferably, the microspheres are present in an amount ranging from about 0.5% to about 40% by weight of the total weight of the composition, more preferably from about 1% to about 30% of the total weight of the composition, more preferably from about 2% to about 20% of the total weight of the composition, and most preferably from about 3% to about 15%, including all ranges and subranges therebetween.

Film Forming Component

According to the present invention, compositions comprising a film forming component are provided. The film forming component comprises at least one film forming agent, perhaps two, three or more film forming agents. In one preferred embodiment, one film forming agent is present. In another preferred embodiment, two film forming agents are present. Any substance capable of forming a film upon application to keratin materials and having a glass transition temperature (Tg) can be used as a film forming agent in accordance with the present invention.

Although not wishing to be bound by any particular theory, it is believed that the film forming component according to the present invention provides a matrix-like environment on or around keratin materials, and that this environment enables the microspheres to remain on or around the keratin materials and to impart their characteristics on the keratin materials for a longer period of time. Thus, according to preferred embodiments of the present invention, sufficient film forming component is present in the invention compositions to improve and/or lengthen the micropsheres' effect on the keratin materials to which the compositions have been applied. It is also believed that the film forming component improves the cosmetic effects of compositions containing microspheres. For example, the film forming component of the present invention reduces tackiness of compositions containing microspheres of the present invention.

According to preferred embodiments, the film forming component comprises at least two film forming agents. In such embodiments, the at least two film forming agents are preferably chosen to create an environment which improves and/or lengthens the micropsheres' effect on the keratin materials to which the compositions of the present invention have been applied, and/or which improves the cosmetic properties the compositions of the present invention.

According to one such embodiment, the at least two film forming agents possess substantially different glass transition temperatures (Tgs). For example, the Tgs of the two film forming agents are separated by at least about 20°C, 30°C, 40°C, 50°C, 60°C, 70°C, 80°C, 90°C, including all ranges and subranges therebetween. Most preferably, one film forming agent has a Tg below about -20°C and the other film forming agent has a Tg above about 0°C.

According to another such embodiment, one of the film forming agents possesses a glass transition temperature (Tg) which is greater than 0°C, while the other film forming agent possesses a Tg which is less than 0°C. Examples of Tgs for film forming agents above 0°C include but are not limited to 10°C, 20°C, 30°C, 40°C, 50°C, 60°C, 70°C, 80°C, 90°C, including all ranges and subranges therebetween. Examples of Tgs for film forming agents below 0°C include but are not limited to -10°C, -20°C, -30°C, -40°C, -50°C, -60°C, -70°C,-80°C, -90°C, including all ranges and subranges therebetween.

According to other preferred embodiments, the Tg of one of the film forming agents is above 25°C and the Tg of the other of the two film forming agent is below 25°C.

According to other preferred embodiments, the film forming component comprises at least one film forming agent. In a particularly preferred embodiment, the Tg of the one film forming agent is less than 0°C. Examples of Tgs for film forming agents below 0°C include but are not limited to -10°C, -20°C, -30°C, -40°C, -50°C, -60°C, -70°C, -80°C, -90°C, including all ranges and subranges therebetween. In such embodiments, the at least one film forming agent is preferably chosen to create an environment which improves and/or lengthens the micropsheres' effect on the keratin materials to which the compositions of the present invention have been applied, and/or which improves the cosmetic properties the compositions of the present invention.

Suitable film forming agents for use in accordance with the present invention include, but are not limited to, polyorganosiloxane containing polymers such as polysilicone polyamides or polysilicone polyurethanes; silicone resins (preferably, MK or MQ resin), silicone/(meth)acrylate copolymers, acrylates/dimethicone copolymers, liquid siloxy silicates and silicone esters such as those disclosed in U.S. Pat. No. 5,334,737, silicone polymers comprising a backbone chosen from vinyl polymers, methacrylic polymers, and acrylic polymers and at least one chain chosen from pendant siloxane groups and pendant fluorochemical groups, such as those disclosed in U.S. Pat. Nos. 5,209,924 and 4,972,037, and WO 01/32737, a copolymer chosen from vinyl-silicone graft copolymers such as those polymers described in U.S. Pat. No. 5,468,477.

Suitable film forming agents for use in accordance with the present invention are also disclosed in U.S. patent application publication no. 2004/0170586.

According to particularly preferred embodiments, at least one of the film forming agents is a polymer prepared by polymerization of monomer(s) selected from the group consiting of acrylates, methacrylates, acrylic acid, methacrylic acid, and mixtures thereof. According to more preferred embodiments, both film forming agents are polymers prepared by polymerization of monomer(s) selected from the group consiting of acrylates, methacrylates, acrylic acid, methacrylic acid, and mixtures thereof.

Suitable examples of such film forming agents include, but are not limited to, water-borne emulsion-polymerized acrylic or vinyl-acrylic polymers that are pressure-sensitive adhesives, ethylene-vinyl acetate emulsions, polyurethane dispersions and natural rubber latex. Specific examples include, but are not limited to, Carbotac 26222 (BF Goodrich), Nacrylic 78-6408 (National Starch and Chemical), Rhoplex N580 (Rohm and Haas), Acronal NV 146CR (BASF), Acronal NV 155CR (BASF), Acronal NV 189CR (BASF), Acronal NV 162CR (BASF), Acronal NN 171CR (BASF), Acronal NA 182CR (BASF), NACRYLIC CP3600 (National Starch), NACRYLIC CP2550 (National Starch), NACRYLIC CP3650 (National Starch), Luphen D259U (BASF), DUROSET C-325 (National Starch), RESYN 1025 (National Starch), and DUROSET E-200 (National Starch).

According to most preferred embodiments, the film forming agent(s) are compatible/miscible with the microspheres, and are compatible/miscible with each other if more than one is present.

According to preferred embodiments, the film forming component is present in the composition in an amount ranging from 0.3% to 50% by weight relative to the total weight of the composition. Preferably, the film forming component is present in an amount ranging from 1.0 to 40% by weight relative to the total weight of the composition, and more preferably from 2% to 30%. One of ordinary skill in the art will recognize that these weight percentages are based on dry weight of the film forming agents, and that the film forming agents of the present invention may be commercially available from suppliers in the form of a dilute solution. The amounts of the film forming agent disclosed herein therefore reflect the weight percent of active material.

According to preferred embodiments where two film forming agents are present, the ratio of the first film forming agent to the second film forming agent is between about 1:10 to 20:1, about 1:10 to 10:1, about 1:5 to 5:1, including all ranges and subranges therebetween.

In accordance with the present invention, the microspheres and film forming component can be added to the composition during preparation simultaneously or separately. According to particularly preferred embodiments of the present invention, the microspheres and film forming component are added simultaneously, most preferably in the form of a composition containing microspheres and a suitable film forming component such as the compositions described in U.S. patent application publication no. 2003/0109630. Suitable examples of compositions containing microspheres and a suitable film forming component Multi-Lok 38-454A (National Starch).

Coloring Agents

According to the present invention, the compositions may optionally comprise at least one coloring agent. Suitable coloring agents include but are not limited to pigments (both goniochromatic and monochromatic), dyes, such as liposoluble dyes, nacreous pigments, and pearling agents. Typically, when the composition contains colorants, it is a coloring composition such as a mascara, lipstick or foundation. Alternatively, when the composition does not contain colorants, it is a clear or transparent composition which can be used as a basecoat (or topcoat) prior to (or after) application of a coloring composition to keratin materials. However, it is possible that topcoats or basecoats could contain colorants.

Representative liposoluble dyes which may be used according to the present invention include Sudan Red, DC Red 17, DC Green 6, β-carotene, soybean oil, Sudan Brown, DC Yellow 11, DC Violet 2, DC Orange 5, annatto, and quinoline yellow. The liposoluble dyes, when present, generally have a concentration ranging up to 20% by weight of the total weight of the composition, such as from 0.0001 % to 6%.

The nacreous pigments which may be used according to the present invention may be chosen from white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride. The nacreous pigments, if present, be present in the composition in a concentration ranging up to 50% by weight of the total weight of the composition, such as from 0.1% to 20%, preferably from 0.1 % to 15%.

The pigments, which may be used according to the present invention, may be chosen from white, colored, inorganic, organic, polymeric, nonpolymeric, coated and uncoated pigments. They may also be monochromatic or goniochromatic (for example, a multilayer interference structure) such as those disclosed in U.S. patent 6,451,294. Representative examples of mineral pigments include titanium dioxide, optionally surface-treated, zirconium oxide, zinc oxide, cerium oxide, iron oxides, chromium oxides, manganese violet, ultramarine blue, chromium hydrate, and ferric blue. Representative examples of organic pigments include carbon black, pigments of D & C type, and lakes based on cochineal carmine, barium, strontium, calcium, and aluminum.

lf present, the pigments may be present in the composition in a concentration ranging up to 50 % by weight of the total weight of the composition, such as from 0,5% to 40%, and further such as from 2% to 30%. ln the case of certain products, the pigments, including nacreous pigments, may, for example, represent up to 50% by weight of the composition.

According to particular aspects of the present invention, the compositions can be in the form of an emulsion. Suitable emulsion forms include but are not limited to oil-in-water, water-in-oil, oil-in-water-in-oil, water-in-oil-in-water and nanoemulsions (emulsions whose oil globules are of very fine particle size, that is to say that they have a number-average size of less than about 100 nanometers (nm)). Emulsions contain at least one oil phase and at least one aqueous phase. Typically speaking, emulsions contain surfactants or surfactant-like materials which provide stability to the emulsions and inhibit de-phasing of the emulsions.

Additional Ingredients

The compositions of the present invention can also comprise any additive usually used in the field under consideration. For example, dispersants, antioxidants, essential oils, preserving agents, fragrances, liposoluble polymers that are dispersible in the medium, fillers, neutralizing agents, cosmetic and dermatological active agents such as, for example, emollients, moisturizers, vitamins, anti-wrinkle agents, essential fatty acids, sunscreens, and mixtures thereof can be added. In particular, the composition may further comprises at least one sunscreen agent, at least one pigment, or mixtures thereof. A non-exhaustive listing of such ingredients can be found in U.S. patent application publication no. 2004/0170586. Further examples of suitable additional components can be found in the other references which have been incorporated by reference in this application, including but not limited to the applications from which this application claims priority. Still further examples of such additional ingredients may be found in the International Cosmetic Ingredient Dictionary and Handbook (9th ed. 2002).

A person skilled in the art will take care to select the optional additional additives and/or the amount thereof such that the advantageous properties of the composition according to the invention are not, or are not substantially, adversely affected by the envisaged addition.

These substances may be selected variously by the person skilled in the art in order to prepare a composition which has the desired properties, for example, consistency or texture.

These additives may be present in the composition in a proportion from 0% to 99% (such as from 0.01 % to 90%) relative to the total weight of the composition and further such as from 0.1 % to 50% (if present).

Needless to say, the composition of the invention should be cosmetically or dermatologically acceptable, i.e., it should contain a non-toxic physiologically acceptable medium and should be able to be applied to the eyelashes of human beings. For the purposes of the invention, the expression "cosmetically acceptable" means a composition of pleasant appearance, odor, feel and/or taste.

In particular, with respect to compositions for application to hair and/or eyelashes, additional optional ingredients include but are not limited to those ingredients set forth in U.S. Patent No. 6,548,051 and U.S. patent application publication nos. 2002/0059941 and 2006/0024255. Examples of such additional ingredients include but are not limited to adhesive particles, non-adhesive particles, fixing polymers, and propellants (for aerosol compostions).

However, in a particularly preferred embodiment of the present invention, a cosmetic or dermatologic composition which is free of, essentially free of or substantially free of non-adhesive particles is provided. By "free of' non-adhesive particles," it is meant that no non-adhesive particles are present in the composition. By "essentially free of non-adhesive particles," it is meant that less than 0.01% non-adhesive particles are present in the composition. By "substantially free of non-adhesive particles," it is meant that less than 0.03% non-adhesive particles are present in the composition. As used herein, a "non-adhesive" particle is a particle that is not coated with an adhesive as described in U.S. Patent No. 6,548,051, U.S. patent application publication no. 2002/0059941 and U.S. patent application publication no. 2006/0024255. More specifically, a non-adhesive particle is a discrete substrate that is not an adhesive and is not coated with an adhesive coating in whole or in part, and does not comprise an adhesive as a core or shell material when in a core/shell structure.

Specific examples of additional ingredients include oils, particularly if the composition is an anhydrous composition or an emulsion. Any oils can be used in accordance with the present invention. The oils can be volatile or non-volatile, silicone-based and/or hydrocarbon-based, etc. Thus, for example, the external oil phase may contain, independently or in combination, volatile silicone oils, non-volatile silicone oils, volatile non-silicone oils and non-volatile non-silicone oils.

In one embodiment, the compositions of the present invention are substantially free of silicone oils (i.e., contain less than about 1 % of silicone oil). In another embodiment, the compositions are substantially free of non-silicone oils (i.e., contain less than about 1% of non-silicone oil). In another embodiment, the compositions are substantially free of non-volatile oils (i.e., contain less than about 1% of non-volatile oil). In yet another embodiment, the compositions are substantially free of volatile oils (i.e., contain less than about 1 % of volatile oil).

According to one embodiment, the oil phase may contain one or more volatile silicone oils. Examples of such volatile silicone oils include linear or cyclic silicone oils having a viscosity at room temperature less than or equal to 6cSt and having from 2 to 7 silicone atoms, these silicones being optionally substituted with alkyl or alkoxy groups of 1 to 10 carbon atoms. Suitable oils that may be used in the invention include octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

**Table 1**

| Compound | Flash Point (°C) | Viscosity (cSt) |
|---|---|---|
| Octyltrimethicone | 93 | 1.2 |
| Hexyltrimethicone | 79 | 1.2 |
| Decamethylcyclopentasiloxane | 72 | 4.2 |
| (cyclopentasiloxane or D5) | | |
| Octamethylcyclotetrasiloxane | 55 | 2.5 |
| (cyclotetradimethylsiloxane or D4) | | |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 | 56 | 1.5 |
| (1.5cSt) from Dow Corning | | |
| PDMS DC 200 (2cSt) from Dow Corning | 87 | 2 |
| PDMS DC 200 (5cSt) from Dow Corning | 134 | 5 |
| PDMS DC 200 (3St) from Dow Corning | 102 | 3 |

Further, a volatile linear silicone oil may be employed in the compositions of the present invention. Suitable volatile linear silicone oils include those described in U.S. patent no. 6,338,839 and WO03/042221. ln one embodiment the volatile linear silicone oil is decamethyltetrasiloxane. ln another embodiment, the decamethyltetrasiloxane is further combined with another solvent that is more volatile than decamethyltetrasiloxane.

The volatility of the solvents/oils can be determined using the evaporation speed as set forth in U.S. patent no. 6,338,839.

Examples of other silicone oils that may be used in the invention include non-volatile linear polydimethylsiloxanes (PDMSs), that are liquid at room temperature; polydimethylsiloxanes comprising alkyl, alkoxy or phenyl groups, which are pendent and/or at the end of a silicone chain, these groups each containing from 2 to 24 carbon atoms; phenylsilicones, for instance phenyl trimethicones, phenyl dimethicones, phenyl trimethylsiloxydiphenylsiloxanes, diphenyl dimethicones, diphenyl methyldiphenyl trisiloxanes and 2-phenylethyl trimethylsiloxysilicates.

According to other preferred embodiments, the oil phase may contain one or more non-silicone volatile oils and may be selected from volatile hydrocarbon oils, alcohols, volatile esters and volatile ethers. Examples of such volatile non-silicone oils include, but are not limited to, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar or Permethyl, the C₈ to C₁₆ branched esters such as isohexyl or isodecyl neopentanoate and their mixtures. Preferably, the volatile non-silicone oils have a flash point of at least 40°C.

**Table 2**

| **Compound** | **Flash Point (°C)** |
|---|---|
| Isododecane | 43 |
| Isohexadecane | 102 |
| Isodecyl Neopentanoate | 118 |
| Propylene glycol n-butyl ether | 60 |
| Ethyl 3-ethoxypropionate | 58 |
| Propylene glycol methylether acetate | 46 |
| Isopar L (isoparaffin C₁₁-C₁₃) | 62 |
| Isopar H (isoparaffin C₁₁-C₁₂) | 56 |

Examples of other non-silicone oils which can be used in the compositions of the present invention include polar oils such as:
- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810, 812 and 818 by the company Dynamit Nobel;
- synthetic oils or esters of formula R₅COOR₆ in which R₅ represents a linear or branched higher fatty acid residue containing from 1 to 40 carbon atoms, including and better still from 7 to 19 carbon atoms, and R₆ represents a branched hydrocarbon-based chain containing from 1 to 40 carbon atoms, including and better still from 3 to 20 carbon atoms, with R₆ + R₇ ≥ 10, such as, for example, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate; and pentaerythritol esters;
- synthetic ethers containing from 10 to 40 carbon atoms;
- C₈ to C₂₆ fatty alcohols, for instance oleyl alcohol; and
- mixtures thereof.

Preferably, the oils, if present, represent from about 5% to about 80% by weight of the total weight of the composition, more preferably from about 10% to about 60% of the total weight of the composition, and most preferably from about 15% to about 50%, including all ranges and subranges therebetween..

Water, when present, preferably represents from about 1% to about 70% by weight of the total weight of the composition, more preferably from about 5% to about 60% of the total weight of the composition, and most preferably from about 10% to about 50%, including all ranges and subranges therebetween.

According to preferred embodiments, methods of improving performance properties of compositions for keratin materials such as, for example, transfer-resistance, long-wear and/or waterproofing, comprising combining in a cosmetic or dermatologic composition a microsphere and a film forming component are provided. The compositions may be applied to keratin materials as needed, preferably once or twice daily, more preferably once daily and then preferably allowed to dry before subjecting to contact such as with clothing or other objects.

According to particularly preferred embodiments, sufficient microspheres are combined with sufficient film forming component such that the performance properties of the compositions are greater than the performance properties of compositions containing either ingredient individually. Thus, in accordance with such particularly preferred embodiments, the compositions for keratin materials contain a transfer-resistance, long-wear and/or waterproofing enhancing effective amount of the microspheres and/or the film forming component.

According to other preferred embodiments, methods of increasing eyelash or hair volume, strength and/or length comprising applying to eyelashes or hair an eyelash or hair volume-, strength and/or length-increasing effective amount of a composition comprising a microsphere and a film forming component are provided. The compositions may be applied to keratin materials as needed, preferably once or twice daily, more preferably once daily and then preferably allowed to dry before subjecting to contact such as with clothing or other objects.

According to yet other embodiments of the present invention, methods of styling a keratin material such as hair or eyelashes comprising applying to the keratin material a keratin material styling effective amount of a composition comprising a microsphere and a film forming component are provided.

According to yet other embodiments of the present invention, methods of restyling, reshaping or repeatedly fixing a keratin material such as hair or eyelashes comprising initially applying to the keratin material a keratin material styling effective amount of a composition comprising a microsphere and a film forming component, and subsequently restyling, reshaping or repeatedly fixing the keratin material without reapplying the composition to the keratin material are provided.

According to preferred embodiments, methods of holding the shape or curl of a keratin material such as hair or eyelashes comprising applying to the keratin material a shape or curl holding effective amount of a composition comprising a microsphere and a film forming component are provided.

According to other embodiments of the present invention, methods of making-up keratin materials such as eyelashes, nails, skin or lips comprising applying an eyelash, nail, skin or lip making-up effective amount of a composition comprising a microsphere and a film forming component, to eyelashes, nails, skin or lips in need of such making-up are provided.

According to yet other embodiments, methods, in particular non therapeutic methods, of treating or caring for keratin materials such as eyelashes, nails, hair, skin or lips by applying compositions of the present invention to the eyelashes, nails, hair, skin or lips in an amount sufficient to treat and/or care for the eyelashes, nails, hair, skin or lips are provided.

According to other preferred embodiments, methods of enhancing the appearance of keratin materials such as eyelashes, nails, hair, skin or lips by applying compositions of the present invention to the eyelashes, nails, hair, skin or lips in an amount sufficient to enhance the appearance of the eyelashes, nails, hair, skin or lips are provided.

In accordance with the preceding preferred embodiments, the compositions of the present invention are applied topically to keratin materials in an amount sufficient to treat, care for and/or make up the keratin materials, or to enhance their appearance. The compositions may be applied to eyelashes as needed, preferably once or twice daily, more preferably once daily and then preferably allowed to dry before subjecting to contact such as with clothing or other objects.

The present invention also envisages kits and/or prepackaged materials suitable for consumer use containing one or more compositions according to the description herein (for example, kits containing (1) a mascara; and (2) a basecoat and/or topcoat). The packaging and application device for any subject of the invention may be chosen and manufactured by persons skilled in the art on the basis of their general knowledge, and adapted according to the nature of the composition to be packaged. Indeed, the type of device to be used can be in particular linked to the consistency of the composition, in particular to its viscosity; it can also depend on the nature of the constituents present in the composition, such as the presence of volatile compounds.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective measurements. The following examples are intended to illustrate the invention without limiting the scope as a result. The percentages are given on a weight basis.

Example 1 - Microspheres
A. Ingredients which can be used to prepare microspheres by suspension free-radical polymerization pursuant to U.S. patent application publication no. 2003/0109630

| | |
|---|---|
| Monomer | 2-ethylhexyl acrylate |
| Initiator | t-butyl peroctoate |
| Solvent | DI water |
| Surfactant | Sodium dodecyl benzene sulfonate |
| Surfactant | Aerosol MA 80-l |
| Stabilizer | Poly(acrylic acid) |
| Chelating agent | Ethylene diamine tetra-acetic acid |
| Base | Ammonium hydroxide |

B. Formulation of Multi-Lok 38-454A

| | |
|---|---|
| Microspheres | National Starch & Chemical |
| Carbotac 26222 | B.F. Goodrich |
| Nacrylic CP3650 | National Starch & Chemical |
| Acrysol ASE-95 | Rohm & Haas |
| Ammonium Hydroxide | - |

### Example 2

### A. Composition (Mousse) preparation:

| Ingredient | Percentage |
|---|---|
| Multi-Lok 38-454A | 2.35% |
| Empicol BSD 52* (surfactant) | 0.19% |
| Dl Water | 91.46% |
| Propellant A-46 (isobutene/propane) | 6.00% |

| | |
|---|---|
| *Empicol BSD 52 contains Sodium laureth-8 sulfate, Sodium laureth sulfate, Magnesium laureth-8 sulfate, Magnesium laureth sulfate, Sodium oleth sulfate, Magnesium oleth sulfate. | |

The aqueous phase was prepared by dissolving surfactant in deionized water under appropriated agitation rate. Then, polymer (optional - not present in Example A) was added and mixed well. Finally, Multi-Lok 38-454A was added dropwise under continued agitation. 94 part of the above mentioned aqueous phase was mixed with 6 part of propellant A-46 into an aerosol bottle. [Can: Exal PAM 2P 8460 N (50 x 125); Valve: YL31974; Actuator: Mars]

### B. Application:

2 g of mousse was applied on ½ of head of damp hair, followed by blow dry.

### C. Evaluation:

The composition provided good hold and reshapability.

### Example 3

The following compositions were prepared in accordance with the procedures set forth in Example 2.

**Formula A**

| Ingredient | Percentage |
|---|---|
| Multi-Lok 38-454A | 2.00% |
| (Acrylic copolymer) | |
| (National Starch) | |
| Empicol BSD 52 | 0.19% |
| Fixate G-100L | 1.50 |
| (Acrylate/allyl methacrylate copolymer) | |
| (NOVEON; Tg: -20-150°C) | |
| DI Water | 90.31 % |
| Propellant A-46 | 6.00% |
| | |

**Formula B**

| Ingredient | Percentage |
|---|---|
| Multi-Lok 38-454A | 2.00% |
| (Acrylic copolymer) | |
| (National Starch) | |
| Empicol BSD 52 | 0.19% |
| Syntran 5760 | 1.41% |
| (Styrene/acrylate/ammonium methacrylate copolymer) | |
| (Interpolymer; Tg: 19°C) | |
| DI Water | 90.40% |
| Propellant A-46 | 6.00% |
| (Isobutane/propane) | |

**Formula C**

| Ingredient | Percentage |
|---|---|
| Multi-Lok 38-454A | 2.00% |
| (Acrylic copolymer) | |
| (National Starch) | |
| Empicol BSD 52 | 0.19% |
| Copolymer 845 | 1.41 % |
| (Vinylpyrrolidone/dimethylaminoethyl methacrylate) | |
| (ISP; Tg: 175°C) | |
| DI Water | 90.40% |
| Propellant A-46 | 6.00% |
| (lsobutane/propane) | |

**Formula D**

| Ingredient | Percentage |
|---|---|
| Multi-Lok 38-454A | 2.00% |
| (acrylic copolymer) | |
| (National Starch) | |
| Empicol BSD 52 | 0.20% |
| Aculyn 88 | 0.75% |
| (Acrylate/steareth-20 methacrylate cross-linked polymer) | |
| (ROHM & HAAS; Tg: 24°C as supplied, and 54°C when hydrolyzed at pH = 7.0) | |
| Dl Water | 91.05% |
| Propellant A-46 | 6.00% |
| (Isobutane/propane) | |

**Formula E**

| Ingredient | Percentage |
|---|---|
| Multi-Lok 38-454A | 1.88% |
| (Acrylic copolymer) | |
| (National Starch) | |
| Empicol BSD 52 | 0.20% |
| Luviset Clear | 0.94% |
| (Vinylpyrrolidone/methacrylaminde/N-vinylimidazole copolymer) | |
| (BASF; Tg: 219°C dried film; 85°C at 52% relative humidity; 51°C at 76% relative humidity) | |
| DI Water | 90.98% |
| Propellant A-46 | 6.00% |
| (Isobutane/propane) | |

These compositions were applied to damp hair, followed by blow drying. Like the composition in Example 2, these compositions provided good hold and reshapability. Also, the compositions provided hair with a smooth, non-tacky feel.

## Claims

1. Composition comprising a cosmetically acceptable medium, an adhesive microsphere, and a film forming component.

2. Composition according to claim 1, wherein the film forming component comprises at least two film forming agents.

3. Composition according to claim 2, wherein the Tgs of the at least two film forming agents are separated by at least about 20°C, in particular by at least 60°C.

4. Composition according to claim 2 or 3, wherein the Tg of one of the two film forming agents is above 25°C and the Tg of the other of the two film forming agents is below 25°C.

5. Composition according to anyone of the preceding claims, wherein the composition comprises from about 0.3% to about 50%, in particular from about 2% to about 30%, of the film forming component on a dry weight basis with respect to the total weight of the composition.

6. Composition according to anyone of claims 2 to 5, wherein the ratio of the first film forming agent to the second film forming agent is 1:10 to 20:1.

7. Composition according to anyone of the preceding claims, wherein the adhesive microsphere is prepared by polymerization of monomer(s) selected from the group consisting of acrylates, methacrylates, acrylic acid, methacrylic acid, acrylamides, acrylonitriles, pyrrolidones, vinyl esters, and mixtures thereof and in particular selected from the group consisting of acrylates, methacrylates, acrylic acid, methacrylic acid, and mixtures thereof.

8. Composition according to anyone of the preceding claims, wherein the adhesive microsphere is prepared by polymerization of ethylhexylacrylate.

9. Composition according to anyone of claims 2 to 8, wherein at least one of the film forming agents is a polymer prepared by polymerization of monomer(s) selected from the group consisting of acrylates, methacrylates, acrylic acid, methacrylic acid, and mixtures thereof.

10. Composition according to anyone of claims 2 to 9, wherein both of the film forming agents are polymers prepared by polymerization of monomer(s) selected from the group consiting of acrylates, methacrylates, acrylic acid, methacrylic acid, and mixtures thereof.

11. Composition according to anyone of the preceding claims, wherein the composition further comprises at least one sunscreen agent, at least one pigment, or mixtures thereof.

12. Composition according to anyone of the preceding claims, wherein the composition is essentially free of non-adhesive particles.

13. Composition according to anyone of the preceding claims, wherein the adhesive microsphere has a mean length of about 30 to about 1000 microns, in particular of about 30 to about 100 microns.

14. Method for styling a keratin material comprising applying to the keratin material a keratin material styling effective amount of a composition according to anyone of previous claims comprising a cosmetically acceptable medium, an adhesive microsphere, and a film forming component.

15. Method for holding the shape or curl of a keratin material comprising applying to the keratin material a shape or curl holding effective amount of a composition according to anyone of previous claims.

16. Method according to claims 14 or 15, wherein the keratin material is hair.

17. Method according to claim 14 or 15, wherein the keratin material is an eyelash.

18. Use of an adhesive microsphere and a film forming component to prepare a cosmetic and/or dermatologic composition for styling a keratin material and/or holding the shape or curl of a keratin material.
